Europäisches Patentamt

European Patent Office

Office européen des brevets

(19)

(11) Publication number: **0 247 674 B1**

(12) **EUROPEAN PATENT SPECIFICATION**

(45) Date of publication of patent specification: **15.07.92**

(51) Int. Cl.5: **C12N 9/48**, C12N 9/64, C07K 15/12, C12N 9/72, C07K 3/12, A61K 37/54, C12N 15/00

(21) Application number: **87200909.7**

(22) Date of filing: **15.05.87**

(54) **Plasminogen activator and thrombolytic composition.**

(30) Priority: **15.05.86 NL 8601240**

(43) Date of publication of application:
**02.12.87 Bulletin 87/49**

(45) Publication of the grant of the patent:
**15.07.92 Bulletin 92/29**

(84) Designated Contracting States:
**AT BE CH DE ES FR GB GR IT LI LU NL SE**

(56) References cited:
**EP-A- 0 092 182**
**EP-A- 0 139 447**

**BIOLOGICAL ABSTRACTS, vol. 83, no. 6, 1987, abstract no. 57611; D.C. STUMP et al.: "Purification and characterization of a novel low molecular weight form of single-chain arokinase-type plasminogen activator", & J. BIOL. CHEM. 261(36), 17120-17126, 1986**

(73) Proprietor: **Leuven Research & Development V.Z.W.**
**Benedenstraat 59A Groot Begijnhof**
**B-3000 Leuven(BE)**

Proprietor: **Collen, Désiré José**
**Schoonzichtlaan 20**
**B-3020 Winksele-Herent(BE)**

(72) Inventor: **Collen, Désiré José**
**Schoonzichtlaan 20**
**B-3009 Winksele-Herent(BE)**

(74) Representative: **Bruin, Cornelis Willem et al**
**OCTROOIBUREAU ARNOLD & SIEDSMA**
**Sweelinckplein 1**
**NL-2517 GK The Hague(NL)**

EP 0 247 674 B1

Rank Xerox (UK) Business Services

CHEMICAL ABSTRACTS, vol. 106, no. 17, 27th April 1987, page 38, abstract no. 131457j, Columbus, Ohio, US; D.C. STUMP et al.: "Comparative thrombolytic properties of single-chain forms of urokinase-type plasminogen activator", & BLOOD 1987, 69(2), 592-6

BIOLOGICAL ABSTRACTS/REVIEWS-REPORTS-MEETINGS, vol. 32, 1987, abstract no. 28903; D.C. STUMP et al.: "Biochemical and biological characterization of a novel low molecular weight form of single chain urokinase-type plasminogen activator", & AMERICAN HEART ASSOCIATION MONOGRAPH(US) 1986, vol. O, no. 124, pII-247

PROTIDES BIOL. FLUIDS, vol. 33, 1985, pages 623-626; A. CORTI et al.: "Purification of a single-chain urokinase precursor from A431 human epidermoid carcinoma cells by monoclonal antibody immunoaffinity chromatography"

CHEMICAL ABSTRACTS, vol. 104, no. 11, 17th March 1986, page 280, abstract no. 84223b, Columbus, Ohio, US; D.C. STUMP et al.: "Purification and characterization of single-chain urokinase-type plasminogen activator from human cell cultures", & J. BIOL. CHEM. 1986, 261(3), 1274-8

CHEMICAL ABSTRACTS, vol. 105, no. 19, 10th November 1986, page 41, abstract no. 164686r, Columbus, Ohio, US; D. COLLEN et al.: "Absence of synergism between tissue-type plasminogen activator (t-PA), single-chain urokinase-type plasminogen activator (scu-PA) and urokinase on clot lysis in a plasma milieu in vitro", & THROMB. HAEMOSTASIS 1986, 56(1), 35-9

## Description

This invention relates to a newly found plasminogen activator and moreover to a method of preparing the same and to a thrombolytic composition containing such plasminogen activator.

It is known that certain enzymatic substances called plasminogen activators may be thrombolytically active in vivo after intravenous administration to man or animal. Their activity is based upon activation of plasminogen, a blood component, and this triggers a chain of reactions which finally lead to dissolution (lysis) of a fibrin-containing blood clot if such clot is present in the blood circulation system.

The total activation mechanism is complicated and may show variations dependent from the type of plasminogen activator that has been used. In this connection, a distinction should be made between two types of plasminogen activator, to wit: a tissue-type plasminogen activator (t-PA) and a urokinase-type plasminogen activator (u-PA) and the latter type may be subdivided into a 2-chain form (indicated herein as urokinase) and a 1-chain form (indicated herein as scu-PA) on the basis of molecular conformation. The activating mechanism of these three plasminogen activators is different.

A tissue-type plasminogen activator (t-PA) may cause activation of plasminogen but substantially only in the presence of fibrin. This means that activation is mainly occurring in the vicinity of blood clots which are dissolved thereby and that practically no activation of plasminogen is occurring in the blood flowing through the vessels. Such a plasminogen activator does not react with antisera against urokinase-type plasminogen activators.

The 2-chain form of u-PA (indicated as urokinase) provides for an efficient activation of plasminogen in blood, both in the presence and in the absence of fibrin. This means that blood clots may be dissolved but also that an activation of the whole fibrinolytic blood system will occur and this may lead to fibrinogen brake down and to a certain bleeding tendency.

The 1-chain form of u-PA (indicated as scu-PA) will activate plasminogen efficiently but only in the presence of fibrin-containing blood clots. Therefore and just like in the case of t-PA, little or no activation of the fibrinolytic system in the flowing blood will occur although the activation mechanism is different and although scu-PA is not related immunologically to t-PA.

Among the three kinds of plasminogen activator, urokinase is used in medical practice already for a long time although its secondary activity is found to be objective. Both other kinds of plasminogen activator are still in a stage of clinical experiments which are far advanced in the case of t-PA.

Urokinase is normally recovered from urine which comprises 40-50 ug urokinase per litre in addition to 10-20 $\mu$g/l of scu-PA (D.C. Stump et al., J.Biol.Chem., 261, 1267-1273 (1986)). Urine is less suitable for recovering scu-PA because the scu-PA therein is easily converted to urokinase but it has been found recently that scu-PA may be recovered in stable condition and in fair yields from the culture fluid of certain cell lines in casu the culture fluid of cell line CALU-3 (Stump et al., J.Biol.Chem. 261, 1274-1278 (1986)).

During further investigations about the recovery of scu-PA from the culture fluids of such cell lines, it has now been found that another product having a molecular weight of about 32,000 and having about the same properties as scu-PA is sometimes spontaneously formed in addition to the scu-PA known so far which has a molecular weight ($M_r$) of about 54,000.

According to electrophoresis tests on a sodium dodecyl sulphate-containing polyacrylamide gel (SDS-PAGE), this newly found product is composed only of a single polypeptide chain which has an $M_r$ of about 32,000, both under reducing and non-reducing conditions. The aminoterminal amino acid sequence is:

Leu-Lys-Phe-Gln-Cys-Gly-Gln-Lys

which corresponds to the amino acid sequence in scu-PA of $M_r$ 54,000, starting from amino acid Leu-144. Further, the product has about the same properties as the already known scu-PA such as the potential to cause lysis of a fibrin-containing blood clot in the blood stream without activation of the fibrinolytic blood system. In view of these facts, the new product may be considered to be a low-molecular form of scu-PA. Therefore, this plasminogen activator will further be indicated as scu-PA-32k and, wherever necessary, the known scu-PA will be indicated as scu-PA-54k.

There are indications that this new product scu-PA-32k will be very useful in thrombolytic compositions, just like the known scu-PA-54k. Further, it seems that scu-PA-32k may be prepared more simply than scu-PA-54k in view of its shorter molecular chain. In this respect, advantage may be made of cell lines or micro organisms which have obtained the capacity to produce such a plasminogen activator by means of genetic manipulation.

Thus, in a first aspect, the invention provides a plasminogen activator (scu-PA-32k) derived from a single-chain urokinase-type plasminogen activator of molecular weight of about 54,000 (scu-PA-54k) and characterized by having:

- a single peptide chain,
- a molecular weight ($M_r$) of about 32,000,

- an aminoterminal amino acid sequence of Leu-Lys-Phe-Gln-Cys-Gly-Gln-Lys,
- said aminoterminal sequence starting at the leucine residu corresponding to position 144 of scu-Pa-54k,
- and a potential to cause lysis of fibrin-containing blood clots in blood without activation of the whole fibrinolytic blood system.

Further, the invention provides a preparation method for scu-PA-32k and a thrombolytic composition which comprises scu-PA-32k as an active ingredient.

It should be noted that urokinase may occur as well in a high molecular form ($M_r$ 54,000) and a low-molecular form ($M_r$ 33,000). The low molecular form is sometimes found in urine and is apparently formed by proteolytic conversion of the high molecular form with the aid of trypsin or plasmin which causes removal of an aminoterminal portion of 135 amino acids. However, this low-molecular product bears no similarity with the newly found scu-PA-32k from a functional point of view.

With regard to the properties of scu-PA-32k the following should still be noted; in laboratory tests, scu-PA-32k is relatively inactive towards low molecular chromogenic substrates which induce a high activity of urokinase. Further, it is capable of activating plasminogen with high affinity and low turnover rate just like scu-PA-54k. By means of plasmin, scu-PA-32k is converted to urokinase which displays full activity towards chromogenic substrates and which is capable of activating plasminogen with low affinity and high turnover rate. Scu-PA-32k has a high fibrinolytic activity on fibrin films (more than 50,000) IU/mg, compared with the international standard for urokinase). If scu-PA-32k is added to a system comprising a blood clot in human blood plasma, then such blood clot is dissolved within a certain concentration area without activity of the fibrinolytic system in the surrounding plasma and without noticeable fibrinogen breakdown. Nevertheless, scu-PA-32k does not have a specific fibrin affinity.

Scu-PA-32k may be prepared in principle in several ways. In the first place, it may be prepared by culturing scu-PA-producing cell lines followed by processing of the culture fluid (a conditioned medium or an extract thereof). The processing step may be effected in several ways, for instance by chromatography on zinc chelate-agarose, chromatography on SP-sephadex, gel filtration, chromatography on insolubilized monoclonal or polyclonal antibodies, or combinations thereof. Under suitable conditions, scu-PA-32k may be obtained as a final product then in addition to the known product scu-PA54k.

The human lung adenocarcinoma cell line CALU-3 (ATCC-HTB-55) has appeared to be the most suitable cell line for production of scu-PA thusfar. This cell line will produce mainly scu-PA-54k but a fair amount of scu-PA-32k may be recovered under suitable conditions from its culture fluid during the processing step. An optimalization of the yield of scu-PA-32k and a conversion of nearly all scu-PA-54k in the culture fluid to scu-PA-32k must be obtainable by means of optimalization of conditions and/or utilization of a suitable protease. Further, cell lines or micro organisms may be found which are capable (either spontaneously or after genetic manipulation) to directly produce human scu-PA-32k whereupon such scu-PA-32k may be recovered in a simple way by culturing such cell lines or micro organisms followed by processing of the culture fluid or of an extract thereof. Compare Examples IV-VI below.

Scu-PA-32k may be combined with a suitable excipient to provide a thrombolytically active medical composition. The excipient may be any conventional medium for this purpose. Further, another plasminogen activator such as t-PA may be used concomitantly with scu-PA-32k, thus creating a possible synergistic effect. The resulting composition has preferably the form of an intravenous infusion fluid since this form has the best chances of efficient results.

The invention is further illustrated by the following examples. Several tests were used therein and the nature of these tests will be explained first.

## TESTS

Test for determining urokinase antigen (ELISA).

Polystyrene microtiter plates were coated with total IgG from a rabbit antiserum raised against urokinase and then washed. Thereupon, a urokinase standard and test samples diluted with a buffer solution of 0.05 M phosphate and 0.1 M NaCl were applied to the wells of the plates. This buffer solution (indicated as PBS-Tween) had a pH of 7.4 and further comprised 0.01% of Tween 80. The contents of the wells were incubated at room temperature for 1 hour. After washing with PBS-Tween, the wells were incubated with a murine monoclonal antibody (4D1-E8) which had been conjugated to horseradish peroxidase by the method of Nakane and Kawaoi (J.Histochem. Cytochem. 22, 1084-1091, (1974)). This monoclonal antibody reacts both with urokinase of molecular weight 54,000 and with urokinase of molecular weight 33,000, thus permitting the detection of both molecular forms of urokinase. After 1 hour incubation at 37°C, the bound IgG was quantitatively determined by addition of o-phenylenediamine and measuring of the absorbance at 492 nm. A linear correlation (r = 0.99) between the absorbance at 492 nm and the concentration of urokinase could be observed in the range between 1 and 10 ng/ml for a molecular weight of 54,000, and moreover a linear correlation could be observed in the range between 0.6 and 6 ng/ml for a molecular weight of 33,000.

A similar test was used for detecting only high molecular urokinase antigen. In this test, the monoclonal antibody 4D1-E8 was substituted by a monoclonal antibody 13B6-A8 which will react with high molecular urokinase but not with low molecular urokinase.

Test on fibrinolytic activity.

The fibrinolytic activity was determined on plasminogen-containing bovine fibrin plates after overnight incubation at 37°C (compare Astrup et al., Arch. Biochem. Biophys. 40, 346-351 (1952)) or by lysis of a fibrin clot according to Stump et al. (J.Biol.Chem., 261, 1267-1273 (1986)).

Determination of molecular weight.

The molecular weights were determined by electrophoresis on a sodium dodecyl sulphate-containing polyacryl amide gel, sometimes with reduction of disulphide bridges by 0.05 M dithioerythritol (DTE). This method will be indicated as SDS-PAGE.

EXAMPLE I

Cell culture.

Human lung adenocarcinoma cells of the type CALU -3 (ATCC cell repository line HTB-55) were used in this example. The cells were cultured in plastics flasks at 37°C in atmospheric air with 5% $CO_2$. The culture medium was a modified Dulbecco's medium including 4.5 g/l glucose and 25 mM Hepes, supplemented with 4 mM glutamine, 0.1% $NaHCO_3$, 1 mM sodium pyruvate, non-essential amino acids and 10% by volume of fetal calf serum. Further, penicillin and streptomycin were added each to a final concentration of 100 IU/ml. Confluency was reached after seven days. The confluent monolayers were washed with a sterile buffer solution, treated for 5 min. with a mixture of trypsin and EDTA and, after decanting, incubated for 30 minutes at 37°C in atmospheric air with 5% $CO_2$. Thereafter the cells were suspended again in Dulbecco's modified medium, subdivided in a ratio of 1:3 and further cultured to confluency.

During harvesting, the confluent monolayers were washed twice with a sterile buffer solution of pH 7.4 and thereafter incubated with 25 ml Eagle's essential medium supplemented with 1 mM glutamine, 0.1% $NaHCO_3$, 1% fungizone, 200 IU/ml penicillin and aprotinin 10 KIU/ml. The culture fluid was harvested at intervals of 1-3 days, centrifuged at 4°C for 30 minutes and then frozen at -20°C until the time of use.

Purification of the culture fluid.

The culture fluid of the CALU-3 cells was purified for obtaining scu-PA by chromatography on zinc chelate-Sepharose, SP-Sephadex and Sephadex G-100.

In the first step, 50 l of culture fluid were passed at a flow rate of 100 ml/hour through a column of zinc chelate-Sepharose equilibrated with a buffer solution of pH 7.5 which included 0.05 M NaCl, 0.05 M $NaH_2PO_4$ and moreover 0.01% Tween 80 and 20 KIU/ml aprotinin. Afterwards, the column was washed with 2 litres of buffer solution and eluted with 4 litres of buffer solution including a linear gradient of 0 to 0.1 M imidazole. Fractions of 20 ml were collected in tubes containing 20 KIU/ml aprotinin and assayed for the presence of urokinase antigen with the ELISA-test. The antigen-containing fractions were pooled, adjusted to a pH of 6.8 and diluted threefold with distilled water.

The pooled eluates of two such purification steps were passed through a column of SP-Sephadex C-50 equilibrated with a buffer solution of pH 6.8 which included 0.05 M NaCl, 0.05 M phosphate and moreover 0.01% Tween 80 and 20 KIU/ml aprotinin. The column was washed with buffer solution and thereafter eluted with buffer solution containing from 0.05 to 0.5 M NaCl. Fractions of 10 ml were collected in tubes containing 50 KIU/ml aprotinin. The fractions containing urokinase antigen were pooled and concentrated to 10 ml by dialysis against solid polyethylene glycol 20,000 after addition of solid KSCN.

The concentrated eluate was passed through a column of Sephadex G-100 superfine equilibrated with a buffer solution of pH 7.5 which included 1.0 M KSCN, 0.01 M $NaH_2PO_4$ and moreover 0.01% Tween 80 and 20 KIU/ml aprotinin. Fractions of 5 ml were collected and tested on total protein by measuring the absorbance at 280 nm. Further, the presence of urokinase antigen was determined with the ELISA-test and the fibrinolytic activity was determined by lysis of a blood clot.

As a result of the third purification step, two different products (indicated as peak I and peak II) were obtained which both contained an urokinase antigen according to the ELISA-test. The yield was 79 ± 11 ug and 56 ± 20 ug per litre of initial culture fluid, respectively. Both products were separately dialyzed against a buffer solution of pH 7.5 which included 0.15 M NaCl, 0.01 M $NaH_2PO_4$ and moreover 0.01% Tween 80 and 20 KIU/ml aprotinin for the removal of KSCN, and thereafter stored in frozen condition at -20°C.

Analysis by SDS-PAGE revealed that the material of peak I migrated as a single band of molecular weight 54,000, both under non-reducing and under reducing conditions. Therefore, this product was normal scu-PA. The material of peak II migrated as a substantially homogeneous band having a molecular weight of about 32,000, both under reducing and under non-reducing conditions. Therefore, this product was low molecular 1-chain-PA or scu-PA-32k.

EXAMPLE II

Characterization of the product.

Upon analysis of scu-PA-32 (obtained as peak II in the preceding example), it appeared to have the following aminoterminal amino acid sequence: Leu-Lys-Phe-Glx-X-Gly-Glx, wherein X = not observed. This corresponds to the amino acid sequence in scu-PA of molecular weight 54,000 which starting with residue 144 is as follows: Leu-Lys-Phe-Gln-Cys-Gly-Gln-Lys. Thus, the polypeptide chain has apparently been split up between units 143 and 144.

Further, the characteristics of scu-PA-32k were very similar to those of scu-PA-54k. The products of peak I and peak II had a specific activity of 35,000 and 71,000 IU/mg resp. on fibrin plates but only 5,000 and 10,000 IU/mg resp. towards the chromogenic substrate S-2244. Both products activated plasminogen with high affinity ($K^m$ = 0.8 uM and 0.9 uM resp.) and a low turnover rate with $k_{cat}$ = 0.009 and 0.0028 $s^{-1}$ resp. Both products were converted to urokinase (2-chain-PA) by means of plasmin and in that case the values for $K_m$ were 5 and 12 uM resp. and the values for $k_{cat}$ were 0.23 and 0.31 $s^{-1}$, resp. Both scu-PA-54k and scu-PA-32k caused lysis of a $^{125}$I-labelled fibrin clot immersed in human blood plasma without any break-down of fibrinogen in the blood plasma taking place. This lysis potential was still present after 48 hours of preincubation in blood plasma. Moreover, the lysis of blood clots occurred without any direct binding of scu-PA-54k or scu-PA-32k to fibrin.

Finally, after intravenous administration to rabbits, scu-PA-54k and scu-PA-32k caused a comparable thrombolysis in vivo of a labelled blood clot in the vena jugularis without any concomitant fibrinogen break-down in the circulating blood. The test animal model used thereby has been described earlier.

EXAMPLE III

Comparison with urokinase.

When tested with SDS-PAGE, the product of peak II appeared to comigrate with urokinase of molecular weight 33,000 from human urine, at least under non-reducing conditions. However, after reduction of disulphide bridges with 0.05 M dithioerytritol, the urokinase of molecular weight 33,000 had been split up into a B-chain of molecular weight 30,000 and an invisible A-chain of molecular weight 3,000 whereas an unchanged molecular weight of 32,000 was shown by the product of peak II.

The amino acid sequence of scu-PA-32k appeared to start at unit 144 of the polypeptide chain, but the amino acid sequence of molecular weight 33,000 urokinase starts already at unit 136 of the polypeptide chain.

Contrary to the material of peak II, urokinase of molecular weight 33,000 and urokinase of molecular weight 54,000 will activate plasminogen with low affinity (high $K_m$) and high turnover rate (high $k_{cat}$). Further, urokinase of molecular weight 33,000 has a high activity on low molecular substrates and its dissolving effect on blood clots, both in vitro and in vivo, is accompanied with fibrinogen break-down in the surrounding or circulating blood plasma.

EXAMPLE IV

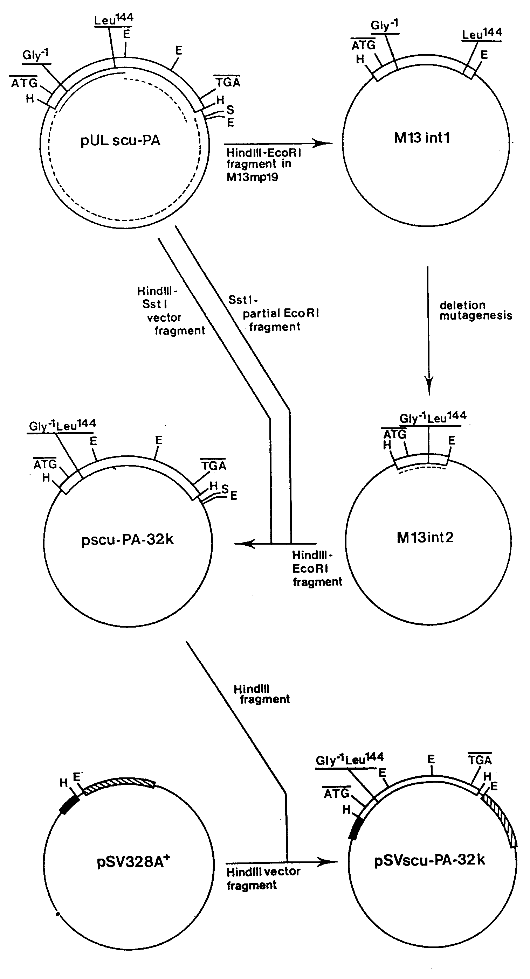

This example relates to cell lines which have obtained the capacity to directly produce scu-PA-32k by means of genetic manipulation. Reference is made to the only figure of the appended drawing which shows the main steps of such genetic manipulation, viz. the construction of cDNA coding for scu-PA-32k and the construction of expression vectors thereof.

In the drawing, the restriction enzymes used (HindIII, EcoRI and SstI) have been indicated by H,E and S, respectively. Start and stop codons are overlined. The codons for amino acids $Gly^{-1}$ and $Leu^{144}$ are underlined and are indicated by the amino acid for which they code. Black boxes indicate the SV40 early promoter/enhancer region, hatched boxes are β-globin gene 3'-sequences, open boxes represent scu-PA cDNA and lines are bacterial sequences.

Several materials were used in the genetic manipulation and the following literature references apply thereto:

- λgt11: Huynn et al, λgt10 and λgt11 DNA Cloning Techniques: A Practical Approach, IRC Press, Oxford, 1984.
- M13mp19 and pUC18: Yannisch-Perron et al, Gene, 33, 103-119 (1985)
- pSV328A⁺: Van Heuvel et al, J.Gen.Virol. 67, 2215-2222 (1986)

- DHFR-deficient CHO cells: Urlaub et al, Proc. Natl. Acad. Sci. U.S.A. 77, 4216-4220 (1980).

The various steps of the genetic manipulation may be explained as follows:

a) oligo(dT)-primed cDNA was prepared from the total RNA of CALU-3 cells and cloned in λ gt11. Several clones were obtained thereby. Three overlapping cDNA clones were spliced together to produce a contiguous cDNA sequence encoding full-length scu-PA. This cDNA was provided with HindIII restriction sites and cloned in pUC18. The resulting clone was called pULscu-PA.

b) a HindIII-EcoRI restriction endonuclease fragment containing 88 base pairs of 5′-untranslated DNA, followed by amino acid codons for $Ser^1$ through $Glu^{163}$ (underlined in the figure) was isolated from pULscu-PA and subcloned in M13mp19. The resulting DNA-intermediate was called M13int1.

c) the intermediate M13int1 was subjected to deletion mutagenesis in order to obtain an intermediate M13int2 having a truncated DNA-sequence. This was effected with the aid of a synthetic oligonucleotide 5′-CTGAAATTTTAAGCCTTTGGAGTC-3′ which was complementary to the amino acid codons for $Asp^{-4}$ through $Gly^{-1}$ and $Leu^{144}$ through $Gln^{147}$ of the scu-PA sequence. The total scu-PA sequence has been disclosed by Holmes et al. in Biochem., 3, 923-929 (1985).

d) a HindIII-EcoRI restriction fragment (broken line) was isolated from M13int2 and substituted for the wild-type DNA sequence in pULscu-PA. This was effected by ligating said fragment into a SstI-partial EcoRI fragment and a HindIII-SstI vector fragment (broken lines) from pULscu-PA. The resulting plasmide was called pscu-PA-32k.

e) a HindIII restriction fragment from pscu-PA-32k, containing the complete scu-PA-32k coding sequence was isolated from pscu-PA-32k and inserted into the HindIII restriction site of the eukaryotic expression vector $pSV328A^+$ resulting in a vector PSVscu-PA-32k.

f) DHFR-deficient Chinese Hamster Ovary cells were transfected with 10 μg pSVscu-PA-32k and 1 μg pSV5DHFR, using the calcium phosphate coprecipitation method. Selection for $DHFR^+$ cells was in Ham's F-12 medium lacking glycine, thymidine and hypoxanthine and supplemented with 8% dialyzed fetal calf serum. Isolated $DHFR^+$ cells were monitored for secretion of urokinase antigen with the aid of the aforementioned ELISA test. One cell line secreting the highest amount of antigen was used for large scale production of the protein.

EXAMPLE V

This example illustrates the production of recombinant scu-PA-32k from the cell line obtained in example IV.

Cell culture.

The cells were grown in roller-bottles in 300 ml MEM α medium (Gibco/BRL, Gaunt, Belgium) supplemented with 10% fetal calf serum and 25 mM Hepes buffer of pH 7.3. At confluency, the medium was replaced by serum free medium consisting of Optimem (Gibco/BRL) supplemented with 10 μg/ml insulin, 5 ug/ml transferrin, 1X non-essential amino acids, 100 U/ml penicillin/streptomycin, 2mM L-Glutamic acid, 5 mM Hepes buffer of pH 7.3, 1 mM Na-pyruvate and 7.5 μg/ml glycine and 20 KIU/ml aprotinin. The conditioned medium was harvested 3 to 4 times after 2 day incubation periods.

Purification of the conditioned medium.

The conditioned medium of the transfected CHO cells was purified for obtaining scu-PA-32k by chromatography on zinc chelate-Sepharose and immunoadsorption on insolubilized monoclonal antibody MA-4D1E8.

13 litres of conditioned medium were passed through a column of zinc chelate-Sepharose, equilibrated with TNTA buffer at a flow rate of 200 ml/h at 4°C. The column was washed with equilibration buffer and eluted with buffer solution containing 50 mM imidazole. The presence of urokinase antigen in the fractions was assayed by the ELISA-test. The antigen-containing fractions were pooled.

The pooled fractions of the first purification step (about 130 ml) were passed at 4°C through an immunoadsorption column of the monoclonal antibody MA-4D1E8 at a flow rate of 10 ml/h. The column was washed with TNTA buffer and then eluted with 2 M KSCN in the same buffer. The fractions containing urokinase antigen were pooled, dialyzed against TNTA buffer, passed over a small benzamidine-Sepharose column (2 ml gel) and washed on a Centricon 30 microconcentrator to remove aprotinin. The product was obtained in a yield of 1.6 mg/l, that is a recovery of 80%. This product was termed recombinant scu-PA-32k or rscu-PA-32k.

EXAMPLE VI

Characterization of rscu-PA-32k.

The product of Example V was subjected to various tests. Upon analysis by SDS-PAGE, the product migrated both under reducing and non-reducing conditions as a main band with $M_r$ 32,000. Moreover, three additional faint bands with $M_r$

33,000-35,000 were observed. Aminoterminal amino acid sequencing revealed a single sequence Leu-..., indicating that the translation product of cDNA was correctly processed and that the occurrence of several $M_r$ forms on SDS-PAGE is probably due to carbohydrate heterogeneity. The product rscu-PA-32k shows a very low activity on chromogenic substrates such as S-2444 (120 IU/mg), a plasminogen-dependent fibrinolytic activity on fibrin plates (specific activity 170,000 IU/mg) and a lack of specific bounding to fibrin. It activates plasminogen directly following Michaelis-Menten kinetics with relatively high affinity ($K_m$ = 2.9 $\mu$M) and a very low turnover rate constant ($k_2$ = 0.0002 $s^{-1}$). The product is converted by plasmin to active two-chain urokinase (rtcu-PA-32k) following Michaelis-Menten kinetics with $K_m$ = 4.3$\mu$M and $k_2$ = 0.62 $s^{-1}$. The resulting rtcu-PA-32k activates plasminogen with $K_m$ = 83 uM and $k_2$ = 4.0 $s^{-1}$.

Both rscu-PA-32k and rtcu-PA-32k cause a similar dose dependent lysis of a [125]I-labelled fibrin clot immersed in human plasma but the single chain form causes less fibrinogen breakdown than the two-chain derivative.

It may be concluded that the functional properties of rscu-PA-32k (expressed with high efficiency) are very similar to those of its natural counterpart mentioned in Example III.

## Claims
**Claims for the following Contracting States : AT, BE, CH, DE, FR, GB, IT, LI, LU, NL, SE**

1. A plasminogen activator (scu-PA-32k) derived from a single-chain urokinase-type plasminogen activator of molecular weight of about 54.000 (scu-PA-54k) and characterized by having:
   - a single peptide chain,
   - a molecular weight ($M_r$) of about 32,000,
   - an aminoterminal amino acid sequence of Leu-Lys-Phe-Gln-Cys-Gly-Gln-Lys,
   - said aminoterminal sequence starting at the leucine residu corresponding to position 144 of scu-Pa-54k,
   - and a potential to cause lysis of fibrin-containing blood clots in blood without activation of the whole fibrinolytic blood system.

2. A plasminogen activator as claimed in claim 1, characterized by a potential to activate plasminogen with high affinity and low turnover rate.

3. A method of preparing a plasminogen activator, characterized by:
   - culturing a cell line or micro organism capable of producing a plasminogen activator as claimed in claim 1 or 2,
   - and recovering a plasminogen activator as claimed in claim 1 or 2 from the culture medium of said cell line or micro organism or from an extract thereof.

4. A method as claimed in claim 3, wherein said plasminogen activator is recovered from the culture fluid or the extract by chromatography on zinc chelate-agarose, chromatography on SP-sephadex, gel filtration, chromatography on insolubilized monoclonal or polyclonal antibodies or combinations thereof.

5. A method as claimed in claim 3 or 4, wherein said plasminogen activator is recovered from the culture medium or the extract of a cell line or micro-organism which has the capacity (either spontaneously or as a result of genetic manipulation) to produce said plasminogen activator directly.

6. A pharmaceutical composition having thrombolytic activity, which comprises the plasminogen activator claimed in claim 1 or 2 as an active ingredient.

7. A method of preparing a pharmaceutical composition having thrombolytic activity, which comprises combining the plasminogen activator claimed in claim 1 or 2 with a suitable excipient.

**Claims for the following Contracting States : ES, GR**

1. A method of preparing a plasminogen activator, comprising the steps of culturing a cell line or micro-organism capable of producing a plasminogen activator and recovering such a plasminogen activator from the culture fluid of that cell line or micro organism or from an extract thereof, characterized by the fact that the cell line or micro organism is capable of producing a plasminogen activator derived from single-chain eurokinase-type plasminogen activator of molecular weight ($M_r$) of about 54,000 (scu-PA-54k) and having:
   - a single peptide chain,
   - a molecular weight ($M_r$) of about 32,000,
   - an aminoterminal sequence of Leu-Lys-Phe-Gln-Cys-Gly-Gln-Lys-,
   - said aminoterminal sequence starting at the leucine residu corresponding to position 144 of scu-PA-54k,
   - and a potential to cause lysis of fibrin-containing blood clots in blood without activation of the whole fibrinolytic blood system,
and that a plasminogen activator of such characteristics is recovered from the culture fluid of that cell line or micro organism or from an extract thereof.

2. A method as claimed in claim 1, wherein said plasminogen activator has a potential to activate plasminogen with high affinity and low turnover rate.

3. A method as claimed in claim 1 or 2, wherein said plasminogen activator is recovered from the culture fluid or the extract by chromatography on zinc chelate-agarose, chromatography on SP-sephadex, gel filtration, chromatography on insolubilized monoclonal or polyclonal antibodies or combinations thereof.

4. A method as claimed in any of claims 1-3, wherein said plasminogen activator is recovered from the culture fluid (or the extract) of a cell line or micro organism which has the capacity (either spontaneously or as a result of genetic manipulation) to produce said plasminogen activator directly.

5. A method of preparing a pharmaceutical composition having thrombolytic activity, characterized by combining a plasminogen activator as resulting from the method of claim 1 with a suitable excipient.

**Revendications**
**Revendications pour les Etats contractants suivantes : AT, BE, CH, DE, FR, GB, IT, LI, LU, NL, SE**

1. Activateur du plasminogène (scu-PA-32k) issu d'un activateur du plasminogène du type urokinase à une seule chaîne, de masse moléculaire d'environ 54 000 (scu-PA-54k), et caractérisé par le fait qu'il présente :
   - une seule chaîne peptidique,
   - une masse moléculaire ($M_r$) d'environ 32 000,
   - une séquence d'acides aminés amino terminale de Leu-Lys-Phe-Gln-Cys-Gly-Gln-Lys,
   - ladite séquence amino terminale démarrant au niveau du résidu leucine correspondant à la position 144 du scu-PA-54k, et
   - un potentiel pour provoquer la lyse de caillots sanguins contenant de la fibrine, dans le sang, sans activation de la totalité du système sanguin fibrinolytique.

2. Activateur du plasminogène selon la revendication 1, caractérisé par un potentiel pour activer le plasminogène avec une affinité élevée et une faible vitesse de renouvellement.

3. Procédé de préparation d'un activateur du plasminogène, caractérisé par :
   - la culture d'une lignée cellulaire ou d'un microorganisme capable de produire un activateur du plasminogène tel que défini à l'une des revendications 1 et 2, et
   - la récupération d'un activateur du plasminogène tel que défini à l'une des revendications 1 et 2 à partir du milieu de culture de ladite lignée cellulaire ou du microorganisme ou à partir d'un extrait de celui-ci.

4. Procédé selon la revendication 3, dans lequel ledit activateur du plasminogène est récupéré à partir du fluide de culture ou de l'extrait par chromatographie sur chélate de zinc-agarose, chromatographie sur SP-Sephadex, filtration sur gel, chromatographie sur des anticorps monoclonaux ou polyclonaux rendus insolubles, ou des combinaisons de celles-ci.

5. Procédé selon l'une quelconque des revendications 3 et 4, dans lequel ledit activateur du plasminogène est récupéré à partir du milieu de culture ou de l'extrait d'une lignée cellulaire ou d'un microorganisme qui présente la capacité (soit spontanément, soit comme résultat d'une manipulation génétique) de produire directement ledit activateur du plàsminogène.

6. Composition pharmaceutique ayant une activité thrombolytique, qui comprend, comme ingrédient actif, l'activateur du plasminogène tel que défini à l'une des revendications 1 et 2.

7. Procédé de préparation d'une composition pharmaceutique ayant une activité thrombolytique, qui comprend l'étape de combinaison de l'activateur du plasminogène tel que défini à l'une des revendications 1 et 2 avec un excipient approprié.

**Revendications pour les Etats contractants suivants : ES, GR**

1. Procédé de préparation d'un activateur du plasminogène, comprenant les étapes de culture d'une lignée cellulaire ou d'un microorganisme capable de produire un activateur du plasminogène et récupération d'un tel activateur du plasminogène à partir du fluide de culture de cette lignée cellulaire ou de ce microorganisme ou à partir d'un extrait de celui-ci, caractérisé par le fait que la lignée cellulaire ou le microorganisme est capable de produire un activateur du plasminogène issu d'un activateur du plasminogène du type urokinase à une seule chaîne, de masse moléculaire ($\underline{M}_r$) d'environ 54 000 (scu-PA-54k), et ayant :
- une seule chaîne peptidique,
- une masse moléculaire ($\underline{M}_r$) d'environ 32 000,
- une séquence amino terminale de Leu-Lys-Phe-Gln-Cys-Gly-Gln-Lys,
- ladite séquence amino terminale démarrant au niveau du résidu leucine correspondant à la position 144 du scu-PA-54k, et
- un potentiel pour provoquer la lyse de caillots sanguins contenant de la fibrine, dans le sang, sans activation de la totalité du système sanguin fibrinolytique,

et par le fait qu'un activateur du plasminogène ayant de telles caractéristiques est récupéré à partir du fluide de culture de cette lignée cellulaire ou de ce microorganisme, ou à partir d'un extrait de celui-ci.

2. Procédé selon la revendication 1, dans lequel ledit activateur du plasminogène présente un potentiel pour activer le plasminogène avec une affinité élevée et une faible vitesse de renouvellement.

3. Procédé selon l'une quelconque des revendications 1 et 2, dans lequel ledit activateur du plasminogène est récupéré à partir du fluide de culture ou de l'extrait par chromatographie sur chélate de zinc-agarose, chromatographie sur SP-Sephadex, filtration sur gel, chromatographie sur des anticorps monoclonaux ou polyclonaux rendus insolubles, ou des combinaisons de celles-ci.

4. Procédé selon l'une quelconque des revendications 1 à 3, dans lequel ledit activateur du plasminogène est récupéré à partir du fluide de culture (ou de l'extrait) d'une lignée cellulaire ou d'un microorganisme qui présente la capacité (soit spontanément, soit comme résultat d'une manipulation génétique) de produire directement ledit activateur du plasminogène.

5. Procédé de préparation d'une composition pharmaceutique ayant une activité thrombolytique, caractérisé par la combinaison d'un activateur du plasminogène tel qu'il résulte du procédé de la revendication 1, avec un exci-

pient approprié.

**Patentansprüche**
**Patenansprüche für folgende Vertragsstaaten :**
**AT, BE, CH, DE, FR, GB, IT, LI, LU, NL, SE**

1. Plasminogenaktivator (scu-PA-32k) abgeleitet von einem einkettigen Plasminogenaktivator vom Urokinasetyp mit Molekulargewicht von etwa 54.000 (scu-PA-54k), dadurch **gekennzeichnet,** daß
- er eine einzelne Peptidkette aufweist,
- er ein Molekulargewicht ($\underline{M}_r$) von etwa 32.000 besitzt,
- er eine aminoendständige Aminosäuresequenz von Leu-Lys-Phe-Gln-Cys-Gly-Gln-Lys aufweist,
- daß die genannte aminoendständige Sequenz an dem der Position 144 von scu-Pa-54k entsprechenden Leucinrest beginnt,
- und daß er das Vermögen besitzt, Lysis von fibrinhaltigen Blutgerinnseln im Blut zu bewirken, ohne Aktivierung des ganzen fibrinolytischen Blutsystems.

2. Plasminogenaktivator nach Anspruch 1, **gekennzeichnet** durch ein Potential zur Aktivierung von Plasminogen mit hoher Affinität und niedriger Umsatzrate.

3. Verfahren zur Herstellung eines Plasminogenaktivators, **gekennzeichnet** durch
- Züchten eines Zellstamms oder Mikroorganismus, der einen Plasminogenaktivator gemäß Anspruch 1 oder 2 zu erzeugen vermag,
- Gewinnen eines Plasminogenaktivators wie in Anspruch 1 oder 2 beschrieben, aus dem Kulturmedium des genannten Zellstamms oder Mikroorganismus oder aus einem Extrakt hiervon.

4. Verfahren nach Anspruch 3, bei welchem der genannte Plasminogenaktivator aus der Kulturflüssigkeit oder dem Extrakt durch Chromatographie auf Zinkgelatagarose, Chromatographie auf SP-Sephadex, Gelfiltration, Chromatographie auf insolubilisierten monoklonalen oder polyklonalen Antikörpern oder Kombinationen hiervon, gewonnen wird.

5. Verfahren nach Anspruch 3 oder 4, bei welchem der genannte Plasminogenaktivator aus dem Kulturmedium oder dem Extrakt eines Zellstamms oder Mikroorganismus gewonnen wird, der (entweder spontan oder als Folge genetischer Manipulation) die Fähigkeit besitzt, den genannten Plasminogenaktivator direkt zu erzeugen.

6. Pharmazeutische Zusammensetzung mit thrombolytischer Aktivität, welche den Plasminogenaktivator nach Anspruch 1 oder 2 als aktiven Bestandteil umfaßt.

7. Verfahren zur Herstellung einer pharmazeutischen Zusammensetzung mit thrombolytischer Aktivität, welches die Kombination des Plasminogenaktivators nach Anspruch 1 oder 2 mit einem geeigneten Excipienten umfaßt.

**Patentansprüche für folgende Vertragsstaaten : ES, GR**

1. Verfahren zur Herstellung eines Plasminogenaktivators, umfassend die Schritte Züchten eines zur Erzeugung eines Plasminogenaktivators geeigneten Zellstamms oder Mikroorganismus und Gewinnen eines derartigen Plasminogenaktivators aus der Kulturflüssigkeit dieses Zellstamms oder Mikroorganismus oder aus einem Extrakt hiervon, dadurch **gekennzeichnet,**
daß der Zellstamm oder Mikroorganismus einen Plasminogenaktivator zu erzeugen vermag, der aus einem einkettigen Plasminogenaktivator vom Eurokinase-Typ mit Molekulargewicht ($M_r$) von etwa 54.000 (scu-PA-54k) abgeleitet ist und
- eine einzelne oder eine einzige Peptidkette aufweist,
- ein Molekulargewicht ($M_r$) von etwa 32.000 besitzt,
- eine aminoendständige Sequenz von Leu-Lys-Phe-Gln-Cys-Gly-Gln-Lys umfaßt,
- wobei die genannte aminoendständige Sequenz an dem der Stellung 144 von scu-PA-54k entsprechenden LeucinRest beginnt,
- und wobei der Plasminogenaktivator das Vermögen besitzt, Lysis von fibrinhaltigen Blutgerinnseln im Blut zu bewirken, ohne Aktivierung des ganzen fibrinolytischen Blutsystems,
und weiter dadurch **gekennzeichnet,** daß ein Plasminogenaktivator mit diesen Eigenschaften aus der Kulturflüssigkeit des genannten Zellstamms oder Mikroorganismus oder aus einem Extrakt hiervon gewonnen wird.

2. Verfahren nach Anspruch 1, bei welchem der genannte Plasminogenaktivator ein Potential zur Aktivierung von Plasminogen mit hoher Affinität und niedriger Umsatzrate besitzt.

3. Verfahren nach Anspruch 1 oder 2, bei welchem der genannte Plasminogenaktivator aus der Kulturflüssigkeit oder dem Extrakt durch Chromatographie an Zinkgelatagarose, Chromatographie an SP-Sephadex, Gelfiltration, Chromatographie an insolubilisierten monoklonalen oder polyklonalen Antikörpern, oder Kombinationen hiervon gewonnen wird.

4. Verfahren nach einem der Ansprüche 1 bis 3, bei welchem der genannte Plasminogenaktivator aus der Kulturflüssigkeit oder dem Extrakt eines Zellstamms oder Mikroorganismus gewonnen wird, der (entweder spontan oder als Folge genetischer Manipulation) die Fähigkeit besitzt, den genannten Plasminogenaktivator direkt zu erzeugen.

5. Verfahren zum Herstellen einer pharmazeutischen Zusammensetzung mit trombolytischer Aktivität, dadurch **gekennzeichnet,** daß man einen nach dem Verfahren gemäß Anspruch 1 erhaltenen Plasminogenaktivator mit einem geeigneten Excipienten kombiniert.